# EUROPEAN PATENT SPECIFICATION

(11) **EP 4 087 642 B1**
(45) Date of publication and mention of the grant of the patent: **11.06.2025**
(21) Application number: 20824256.0
(22) Date of filing: 16.12.2020
(51) Int. Cl.: A61N 1/05

(54) **METHOD FOR PRODUCING AN IMPLANTABLE ELECTRODE DEVICE**
VERFAHREN ZUR HERSTELLUNG EINER IMPLANTIERBAREN ELEKTRODENVORRICHTUNG
PROCÉDÉ DE FABRICATION D'UN DISPOSITIF D'ÉLECTRODE POUVANT ÊTRE IMPLANTÉ

(30) Priority: 08.01.2020 EP 20150782
(43) Date of publication of application: 16.11.2022
(73) Proprietor: BIOTRONIK SE & Co. KG, 12359 Berlin (DE)
(72) Inventor: RUMP, Jens, 12049 Berlin (DE); BUCHNER, Dagmar, 10245 Berlin (DE)
(74) Representative: Biotronik Corporate Services SE
(86) International application number: PCT/EP2020/086462
(87) International publication number: WO 2021/139984

(56) References cited:
- WO-A1-2010/099158
- US-A- 5 524 337
- US-A1- 2008 046 051
- US-A1- 2014 155 968
- US-A1- 2017 250 157

## Description

The present invention relates to a method for producing an implantable electrode device according to claim 1.

In such a method, an electrically insulating support and at least one electrically conductive electrode element are provided in order to attach the at least one electrically conductive electrode element to the support.

By means of such an electrode device, a stimulation signal may be emitted when implanted in a patient, for example to cause spinal nerve stimulation. For this purpose, the electrode device may be introduced into the epidural space near the spinal cord of the spine, for example, in order to achieve nerve stimulation of the spinal cord by introducing electrical stimulation energy. However, such an electrode device may also be used for cardiac stimulation, for example together with a pacemaker system or a defibrillation system.

Electrode devices used, for example, for spinal nerve stimulation may be in the form of isodiametric electrodes formed with an approximately circular cross-section, a diameter usually less than 2 mm and ring electrodes arranged thereon, or in the form of so-called paddle electrodes with a flattened end formed on an electrode body, on which a plurality of electrode elements are usually arranged. While isodiametric electrodes may be easily implanted percutaneously and require a small implantation space, paddle electrodes may have improved efficiency due to the fact that an electrode array formed on the flattened end may have a directional characteristic that allows targeted delivery of stimulation energy.

A challenge with such paddle electrodes, however, is to attach the electrode arrangement to the flattened end of the paddle electrode in a simple, reproducible and exactly placeable manner, if possible in a way that may be easily automated.

Designs of paddle electrodes are known, for example, from US 6 895 283 and US 9 561 363.

The object of the present invention is to provide a method for producing an implantable electrode device and an implantable electrode device, which enable a simple and automatable manufacture with the possibility for exact placement of one or more electrode elements.

This object is achieved by subject matter comprising the features of claim 1.

Accordingly, to attach the at least one electrode element to the support, the at least one electrode element and/or the support are heated and the at least one electrode element is pressed against the support.

Attaching the at least one electrode element to the support is done by heating the electrode element and/or the support. The support is made, for example, of a thermoplastic, for example a material comprising polyurethane, so that the support becomes soft when heated and the at least one electrode element may thus be pressed at least partially into the support, thereby forming an integrally bonded connection to the support. The attachment of the at least one electrode element may thus be carried out in a method step that may be easily automated, without the need to additionally produce, for example, an adhesive bond or the like by means of an adhesive for fixing the at least one electrode element to the support.

The material of the support may be heated by heating the at least one electrode element before attaching it to the support. When the at least one electrode element is attached to the support, heat is transferred from the electrode element to the support and heats the support so that the support, which is preferably made of a thermoplastic, becomes soft and thus enters into an integral bond with the at least one electrode element.

In addition or alternatively, the support may also be heated so that the support is heated by a heat source in addition or alternatively to a heat transfer from the electrode element to the support in order to attach the at least one electrode element to the support.

For the attachment of the at least one electrode element, in one embodiment the at least one electrode element and/or the support are heated to a temperature which is at or near the melting temperature of the support material, for example a temperature greater than 100°C, preferably greater than 150°C, more preferably greater than 180°C, for example 190°C. The heating may be carried out, for example, in a furnace in which the at least one electrode element and/or the support to be heated are placed. Alternatively, a locally acting heat source may be used to heat the at least one electrode element or the support. The at least one electrode element may then be placed on the support for example in an automated manner, for example using a robot, with the placement being carried out by pressing the at least one electrode element against the support and thus without any further method step for fastening the at least one electrode element to the support.

The support may be made of a thermoplastic material, for example a polyurethane material, by means of a film with a thickness between 0.1 mm and 0.5 mm, for example 0.2 mm. For example, the support may have a surface area of between 4 mm by 20 mm and 10 mm by 100 mm.

Each electrode element, for example, may have a thickness between 0.05 mm and 0.2 mm and is made of a metal material, for example a platinum material. For example, the electrode element may have a surface area of between 1 mm by 2 mm and 3 mm by 4 mm, for example 2 mm by 3 mm.

In one embodiment, the at least one electrode element is placed with an underside against an upper side of the support. The at least one electrode element may be pressed against the support in such a way that an upper side of the at least one electrode element is flush with the upper side of the support. In another embodiment, however, the at least one electrode element may also be attached to the support in such a way that the at least one electrode element protrudes from the support and thus protrudes with respect to the upper side of the support. The indentation depth to which the at least one electrode element is pressed into the support may be selected in principle, for example, depending on a desired radiation characteristic for the delivery of stimulation energy via the electrode element.

In one embodiment, an electrical supply line is connected to the at least one electrode element before the at least one electrode element is attached to the support. In this embodiment of the method, the supply line connected to the at least one electrode element is preferably placed on the support together with the at least one electrode element and is pressed into the support as the at least one electrode element and/or the support are heated, so that the supply line connected to the at least one electrode element is embedded in the support at least in a region below the electrode element.

In one embodiment, the supply line connected to the at least one electrode element is embedded in the support along the course of said line in the region of the support.

In one embodiment, the electrical supply line is designed as a wire or cord.

In one embodiment, the at least one electrode element has at least one fastening element which is pressed into the support when the at least one electrode element is attached. The at least one fastening element may, for example, protrude in the manner of a tab from an underside of the electrode element facing the support and, when the at least one electrode element is placed, comes into engagement with the support so that an additional form-fitting connection between the at least one electrode element and the support is created via the at least one fastening element and the hold of the at least one electrode element on the support is improved.

The at least one fastening element may, for example, have a hook shape, for example in the form of a barb, wherein, additionally or alternatively, for example, an opening may be formed in the at least one fastening element, with which the material of the support may engage when the at least one electrode element is placed, so that a firm hold of the at least one electrode element on the support is created.

In one embodiment, the at least one electrode element has a pin which protrudes from an underside of the electrode element facing the support and is pressed into the support when the at least one electrode element is placed. When the at least one electrode element is placed, the pin penetrates the support in such a way that the pin is accessible on an underside facing away from the upper side of the support, which makes it possible to connect an electrical supply line via the pin to the at least one electrode element after the at least one electrode element has been placed. The connection of the supply line may be made here in the region of the underside of the support, so that the supply line is located on the underside of the support and thus not on the side of the electrode device through which stimulation energy is emitted during operation.

In one embodiment, the support is at least partially surrounded by an encasement after the attachment of the at least one electrode element, for example by overmoulding the support in some sections with the material of the encasement. The encasement may, for example, be made of a silicone material, and, for example, the support may be completely encased, except for the surfaces of the electrode elements attached to the support which are to be exposed outwardly in order to release stimulation energy during operation of the electrode device.

In one embodiment, a plurality of electrode elements are attached to the support. The electrode elements may be attached to the support to form a regular arrangement, for example in the manner of a matrix, with, for example, a number of rows of electrode elements possibly being formed on the support. For example, two rows of electrode elements may be attached to the support, in which rows there are arranged electrode elements next to each other.

An implantable electrode device has an electrode body connectable to a generator and an electrode end connected distally to the electrode body. The electrode end comprises a support and a plurality of electrode elements attached to the support, the electrode elements being attached to the support by the method described above.

The advantages and advantageous embodiments described above for the method may be applied analogously to the electrode device, and therefore reference should be made in full to the above.

Such an electrode device realises in particular a paddle electrode which may be implanted, for example, for spinal nerve stimulation in an epidural space in the region of the spine and thus adjacently to the spinal cord, in order to effect nerve stimulation in a targeted manner in the region of the spinal cord.

The concept underlying the invention is explained in more detail below with reference to the embodiments shown in the figures, in which:
- Fig. 1: shows a view of an electrode device connected to a generator in the implanted state in the region of the spine of a patient;
- Fig. 2: shows a view of the electrode device in the epidural space in the region of the spine;
- Fig. 3: shows a view of a flattened end of the electrode device;
- Fig. 4A: shows a schematic view of the process of attaching an electrode element to a support;
- Fig. 4B: shows a view of the electrode element in a position attached to the support;
- Fig. 5: shows a view of another embodiment of an electrode element;
- Fig. 6: shows a view of another embodiment of an electrode element;
- Fig. 7: shows a view of another embodiment of an electrode element;
- Fig. 8: shows a view of the electrode element according to Fig. 7 in a position placed on the support; and
- Fig. 9: shows a view of the arrangement according to Fig. 4B, with an additional encasement to enclose the support.

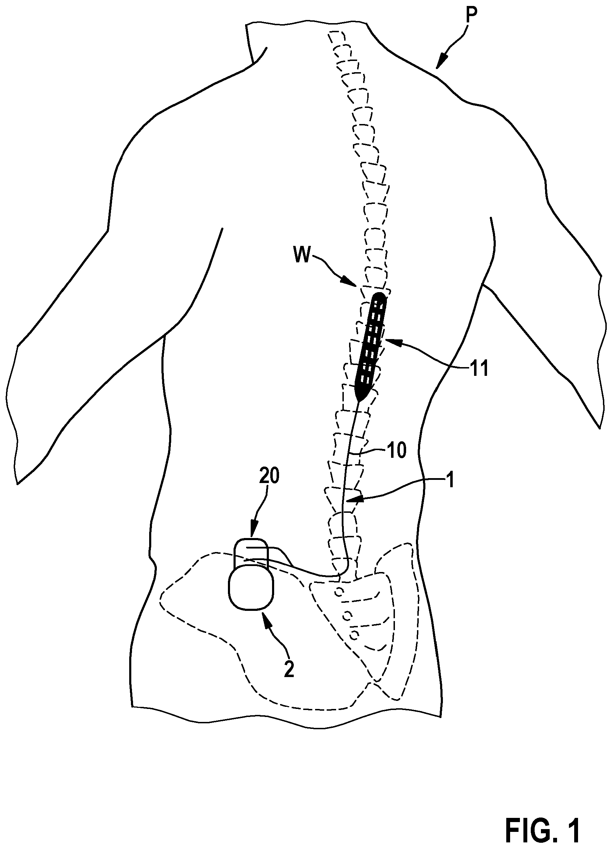

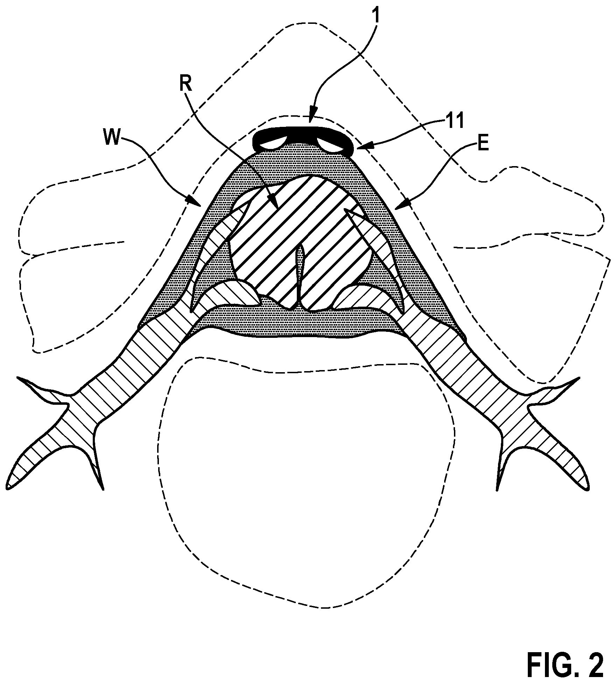

An electrode device 1 shown in Fig. 1 and 2 in one embodiment is realised as a so-called paddle electrode and has an electrode body 10 and an electrode end 11 connected to the electrode body 10, to which electrode end there are attached a plurality of electrode elements for emitting stimulation energy in the region of the spine W of a patient P.

The electrode device 1 is connected by a proximal end of the electrode body 10 to a terminal block 20 of a generator 2, via which stimulation energy may be delivered to the electrode device 1 and radiated via the electrode arrangement arranged at the electrode end 11 for stimulation of the spinal cord R in the region of the spine W.

As may be seen from the sectional view according to Fig. 2, in the embodiment shown, the electrode device 1 is implanted with the electrode end 11 in the epidural space E in the region of the spine W of the patient P in such a way that the electrode end 11 is located in the region of the spinal cord R and may thus introduce stimulation energy in a directed manner into the spinal cord R in order to cause nerve stimulation in the region of the spinal cord R.

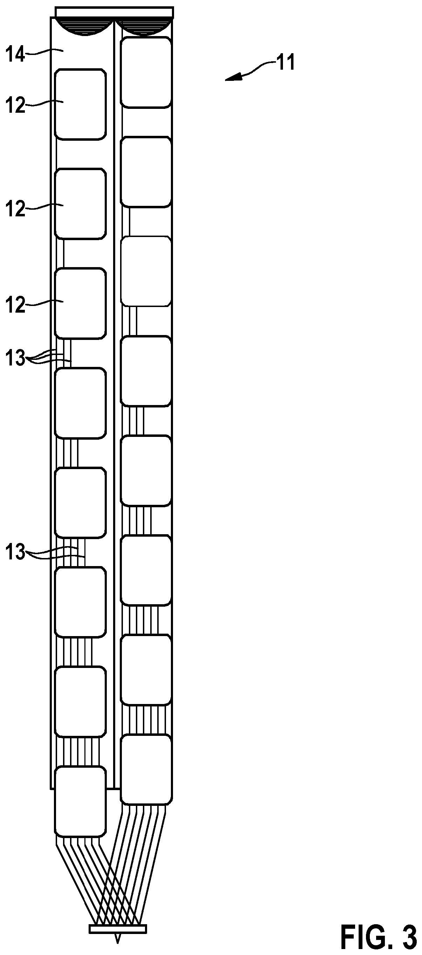

While the electrode body 10, for example, has a circular (isodiametric) cross-section, the electrode device 1 is flattened in the region of the electrode end 11 and, as may be seen in Fig. 3, carries a plurality of electrode elements 12 there, which may be arranged in two rows next to each other and are placed in relation to each other in such a way that stimulation energy may be fed in a directed manner, for example into the spinal cord R of a patient P.

As further shown in Fig. 3, each electrode element 12 is connected to a supply line 13, and each electrode element 12 may be connected to the generator 2 via an associated supply line 13 and may thus be supplied with stimulation energy via the generator 2 to emit an electrical signal. The supply lines 13 are routed together as a cable harness in the electrode body 10 in an encapsulated manner to the generator 2.

The electrode elements 12 are arranged on a support 14, but are exposed with a surface facing outwardly and may therefore come into contact with surrounding tissue when the electrode device 1 is implanted in a patient.

For the fastening of the electrode elements 12 to the support 14, there is a desire for an automatable, simple production process that allows the exact placement of the electrode elements 12 on the support 14.

To this end, it is proposed here to attach the electrode elements 12 to the support 14 by heating the support 14 and/or the corresponding electrode element 12 so that the electrode element 12 in question may be pressed into the support 14 and may form an integrally bonded connection to the support 14.

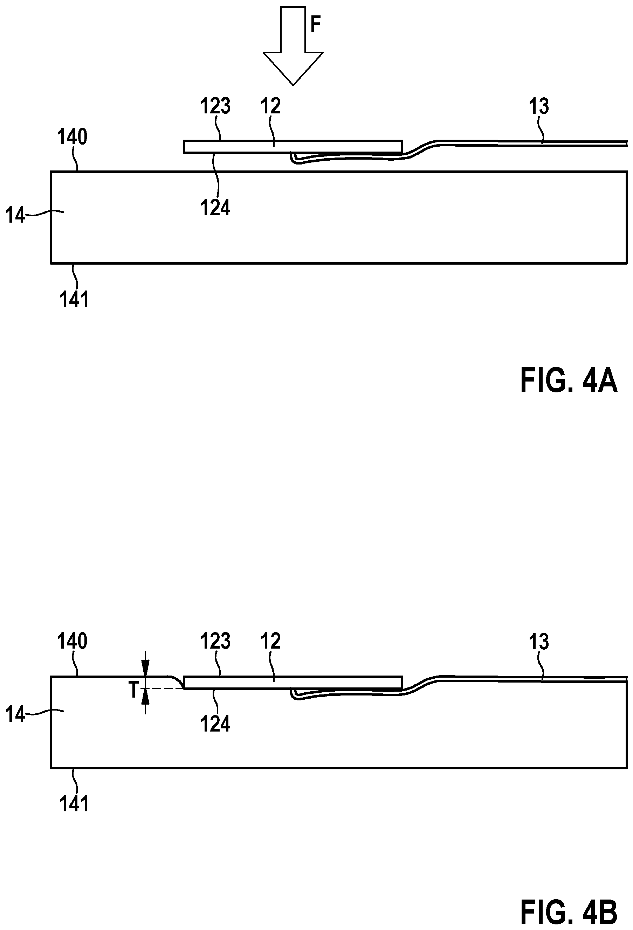

This is illustrated in Fig. 4A and Fig. 4B. For example, an electrode element 12 to be connected to the support 14 may be heated to a temperature at or near the melting temperature of the material of the support 14, which is made for example of a thermoplastic material, for example a polyurethane material, so that when the electrode element 12 is placed, the support 14 is melted in some regions and the electrode element 12 may thus be pressed into the support 14, so that an integral bond is created between the electrode element 12 and the support 14.

In the embodiment shown in Fig. 4A and 4B, the electrode element 12 is placed in a joining direction F against an upper side 140 of the support element 14 in such a way that - as may be seen in Fig. 4B - an upper side 123 of the electrode element 12 is flush with the upper side 140. The indentation depth T to which the electrode element 12 is pressed into the support 14, thus corresponds to the thickness of the electrode element 12.

It should be noted that the upper side 123 of the electrode element 12 does not necessarily have to be flush with the upper side 140 of the support 14. The electrode element 14 may also be placed on the support 14 in such a way that the upper side 123 of the electrode element 12 for example protrudes relative to the upper side 140 of the support 14. In general, the electrode element 12 may be placed on the support 14 with regard to the indentation depth T in such a way that a radiation characteristic for radiating electrical signals via the electrode element 12 is optimised.

To attach the electrode element to the support 14, the electrode element 12 may be heated and brought to a temperature at or near the melting temperature of the material of the support 14, for example. Additionally or alternatively, the support 14 may also be heated. For example, to attach the electrode element 12 to the support 14, the electrode element 12 and/or the support 14 may be heated to a temperature higher than 100°C, for example higher than 150°C, preferably higher than 180°C, for example 190°C.

In the embodiment shown in Fig. 4A and 4B, a supply line 13 is connected to the electrode element 12 before the electrode element 12 is attached to the support 14. In the example shown here, the supply line 13 is connected to an underside 124 of the electrode element 12 facing away from the upper side 123, so that when the electrode element 12 is placed on the support 14, the supply line 13 is located in some sections below the electrode element 12 and is pressed into the material of the support 14 together with the electrode element 12. The supply line 13 is thus embedded below the electrode element 12 in the support 14, so that the point of connection of the supply line 13 to the electrode element 12 is encased by the support 14 and the electrical connection between the supply line 13 and the electrode element 12 is thus secured.

In the embodiment shown in Fig. 4A and 4B, the electrode element 12 forms a flat, for example rectangular or round surface element, which, with the upper side 123 in the attached position, points outwards and may emit electrical signals via the upper side 123 for stimulation, for example in the region of the spinal cord R of a patient P.

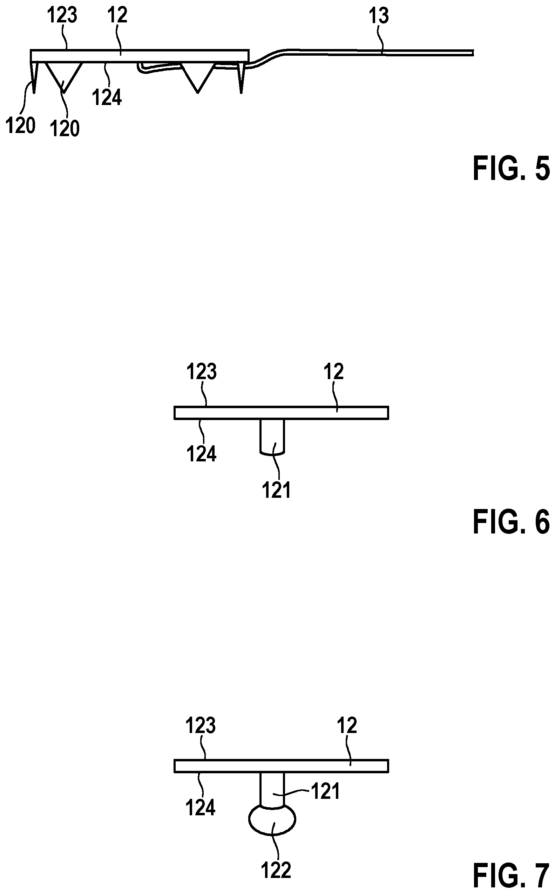

As shown in an embodiment in Fig. 5, the electrode element 12 may have fastening elements 120 in the form of tabs protruding from the underside 124, which serve to improve the mechanical connection of the electrode element 12 to the support 14. When the electrode element 12 is attached to the support 14, the fastening elements 120 come into form-fitting engagement with the support 14 and thus create a form fit between the electrode element 12 and the support 14, so that above this the hold of the electrode element 12 on the support 14 is improved.

A plurality of fastening elements 120 can, for example, be formed along the outer peripheral edge of the electrode element 12 at a distance from each other on the electrode element 12.

The fastening elements 120 can, for example, have a hook shape, for example in the form of barbs. In addition or alternatively, openings may be formed on the fastening elements 120, in which openings the material of the support 14 may engage when the attachment is made, so that a form-fit hold of the electrode element 12 on the support 14 is further improved.

In another embodiment, shown in Fig. 6, a pin 121 is formed on the underside 124 of the electrode element 12, which pin penetrates the support 14 when the electrode element 12 is placed and thus becomes accessible on an underside 141 of the support 14 facing away from the upper side 140, so that a supply line 13 may be connected to the pin 121.

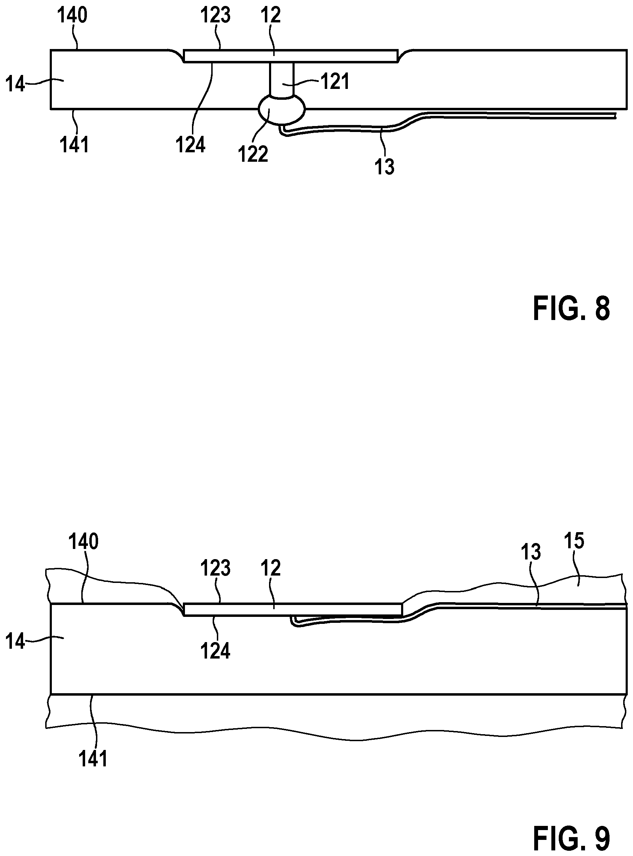

In a more detailed embodiment, shown in Fig. 7, a head 122 may be formed on the pin 121, which head forms an undercut, so that, via the pin 121, the mechanical hold of the electrode element 12 on the support 14 is also improved since a form fit with the support 14 is formed via the head 122, as shown in Fig. 8. A supply line 13 may also be connected to the head 122, as shown in Fig. 8.

Via the pin 121 - in the embodiments of the electrode element 12 according to Figs. 6 and 7 - a supply line 13 may be connected after attaching the electrode element 12 to the support 14. In this case, the supply line 13 may be laid in the region of the underside 141 and thus on a rear side of the support 14, which faces away from the side of the electrode device 1 through which energy is radiated during operation. This may simplify the laying of the supply lines 13 to the different electrode elements 12.

After attaching (all) the electrode elements 12 to the support 14, the support 14 may be surrounded for example by an encasement 15, for example by overmoulding the support 14, as shown in Fig. 9. The encasement 15 may, for example, be made of a silicone material, and the support 14 may be completely surrounded by the material of the encasement 15, but the electrode elements 12 attached to the support 14 are exposed outwardly. By means of the encasement, the electrode device 1 may thus be enclosed and encapsulated in the region of the electrode end 11.

The concept underlying the invention is not limited to the embodiments described above, but may also be realised in other ways, as long as they fall within the scope of the appended claims.

The electrode device may be used for spinal nerve stimulation, but also for other stimulation, for example cardiac stimulation.

One or more electrode elements may be arranged on the support, and - if a plurality of electrode elements are used - an arrangement of electrode elements may be created, by means of which a desired directional characteristic may be provided.

### LIST OF REFERENCE SIGNS

- 1: Implantable electrode device
- 10: Electrode body
- 11: Electrode end
- 12: Electrode element
- 120: Fastening element
- 121: Pin
- 122: Head
- 123: Upper side
- 124: Underside
- 13: Supply line
- 14: Support
- 140: Upper side
- 141: Underside
- 15: Encasement
- 2: Generator
- 20: Terminal block
- E: Epidural space
- F: Joining direction
- O: Surface
- P W: Patient
- R: Spinal cord
- T: Depth Spine

## Claims

1. A method for producing an implantable electrode device (1) comprising an electrode body (10) connectable to a generator (2) and an electrode end (11) distally connected to the electrode body (10), the electrode end (11) comprising an electrically insulating support (14) and at least one electrode element (12) attached to the support (14), wherein the support (14) comprises at least one flat surface, the method comprising the steps of: providing an electrically insulating support (14), providing the at least one electrically conductive electrode element (12), and attaching the at least one electrode element (12) to the at least one flat surface of the support (14), wherein for attaching the at least one electrode element (12) to the support (14), the at least one electrode element (12) and/or the support (14) are heated and the at least one electrode element (12) is pressed against the support (14).

2. The method according to claim 1, wherein the support (14) is made of a thermoplastic.

3. The method according to claim 1 or 2, wherein the support (14) is made of a material comprising polyurethane.

4. The method according to one of claims 1 to 3, wherein for attaching the at least one electrode element (12) to the support (14), the at least one electrode element (12) and/or the support (14) are heated to a temperature greater than 100°C, preferably greater than 150°C, more preferably greater than 180°C.

5. The method according to one of the preceding claims, wherein the at least one electrode element (12) is placed on an upper side (140) of the support (14).

6. The method according to claim 5, wherein the at least one electrode element (12) is pressed against the support (14) in such a way that an upper side (123) of the at least one electrode element (12) is flush with the upper side (140) of the support (14).

7. The method according to one of the preceding claims, wherein an electrical supply line (13) is connected to the at least one electrode element (12) before the at least one electrode element (12) is attached to the support (14).

8. The method according to claim 7, wherein the supply line (13) connected to the at least one electrode element (12) is attached to the support (14) together with the at least one electrode element (12).

9. The method according to one of the preceding claims, wherein the at least one electrode element (12) has at least one fastening element (120) which is pressed into the support (14) when the at least one electrode element (12) is attached to the support (14).

10. The method according to one of the preceding claims, wherein the at least one electrode element (12) has a pin (121) which, when the at least one electrode element (12) is attached to the support (14), is pressed into the support (14) in such a way that the pin (121) penetrates an underside (141) facing away from an upper side (140) of the support (14).

11. The method according to claim 10, wherein after the at least one electrode element (12) has been attached to the support (14), an electrical supply line (13) is connected to the pin (121).

12. The method according to one of the preceding claims, wherein the support (14) is at least partially surrounded by an encasement (15) after the application of the at least one electrode element (12).

13. The method according to claim 12, wherein the encasement (15) consists of a silicone material.

14. The method according to one of the preceding claims, wherein a plurality of electrode elements (12) are attached to the support (14).

15. An implantable electrode device (1) comprising an electrode body (10) connectable to a generator (2) and an electrode end (11) distally connected to the electrode body (10), wherein the electrode end (11) comprises a support (14) having at least one flat surface and a plurality of electrode elements (12) attached to the at least one flat surface of the support (14), wherein the electrode elements (12) are attached to the support (14) by the method according to one of claims 1 to 14.

## Patentansprüche

1. Verfahren zur Herstellung einer implantierbaren Elektrodenvorrichtung (1), einen Elektrodenkörper (10), der mit einem Generator (2) verbindbar ist, und ein Elektrodenende (11), das distal mit dem Elektrodenkörper (10) verbunden ist, umfassend, wobei das Elektrodenende (11) einen elektrisch isolierenden Träger (14) und mindestens ein Elektrodenelement (12), das am Träger (14) befestigt ist, umfasst, wobei der Träger (14) mindestens eine flache Oberfläche umfasst, das Verfahren folgende Schritte umfassend: Bereitstellen eines elektrisch isolierenden Trägers (14), Bereitstellen des mindestens einen elektrisch leitenden Elektrodenelements (12) und Befestigen des mindestens einen Elektrodenelements (12) an der mindestens einen flachen Oberfläche des Trägers (14), wobei zum Befestigen des mindestens einen Elektrodenelements (12) an dem Träger (14) das mindestens eine Elektrodenelement (12) und/oder der Träger (14) erwärmt werden und das mindestens eine Elektrodenelement (12) gegen den Träger (14) gepresst wird.

2. Verfahren nach Anspruch 1, wobei der Träger (14) aus einem Thermoplast hergestellt ist.

3. Verfahren nach Anspruch 1 oder 2, wobei der Träger (14) aus einem Material hergestellt ist, das Polyurethan umfasst.

4. Verfahren nach einem der Ansprüche 1 bis 3, wobei zum Befestigen des mindestens einen Elektrodenelements (12) an dem Träger (14) das mindestens eine Elektrodenelement (12) und/oder der Träger (14) auf eine Temperatur von über 100 °C, bevorzugt über 150 °C, besonders bevorzugt über 180°C erwärmt wird.

5. Verfahren nach einem der vorstehenden Ansprüche, wobei das mindestens eine Elektrodenelement (12) an einer Oberseite (140) des Trägers (14) platziert wird.

6. Verfahren nach Anspruch 5, wobei das mindestens eine Elektrodenelement (12) in der Art und Weise gegen den Träger (14) gepresst wird, dass eine Oberseite (123) des mindestens einen Elektrodenelements (12) bündig mit der Oberseite (140) des Trägers (14) ist.

7. Verfahren nach einem der vorstehenden Ansprüche, wobei eine elektrische Zuleitung (13) an das mindestens eine Elektrodenelement (12) angeschlossen wird, bevor das mindestens eine Elektrodenelement (12) an dem Träger (14) befestigt wird.

8. Verfahren nach Anspruch 7, wobei die Zuleitung (13), die an das mindestens eine Elektrodenelement (12) angeschlossen ist, zusammen mit dem mindestens einen Elektrodenelement (12) an dem Träger (14) befestigt wird.

9. Verfahren nach einem der vorstehenden Ansprüche, wobei das mindestens eine Elektrodenelement (12) mindestens ein Befestigungselement (120) aufweist, das in den Träger (14) gepresst wird, wenn das mindestens eine Elektrodenelement (12) an dem Träger (14) befestigt wird.

10. Verfahren nach einem der vorstehenden Ansprüche, wobei das mindestens eine Elektrodenelement (12) einen Stift (121) aufweist, der, wenn das mindestens eine Elektrodenelement (12) an dem Träger (14) befestigt wird, auf die Art und Weise in den Träger (14) gepresst wird, dass der Stift (121) eine Unterseite (141) durchdringt, die von einer Oberseite (140) des Trägers (14) weg weist.

11. Verfahren nach Anspruch 10, wobei nach dem Befestigen des mindestens einen Elektrodenelements (12) an dem Träger (14) eine elektrische Zuleitung (13) an den Stift (121) angeschlossen wird.

12. Verfahren nach einem der vorstehenden Ansprüche, wobei der Träger (14) nach dem Aufbringen des mindestens einen Elektrodenelements (12) zumindest teilweise von einer Ummantelung (15) umgeben wird.

13. Verfahren nach Anspruch 12, wobei die Ummantelung (15) aus einem SilikonMaterial besteht.

14. Verfahren nach einem der vorstehenden Ansprüche, wobei eine Vielzahl von Elektrodenelementen (12) an dem Träger (14) befestigt werden.

15. Implantierbare Elektrodenvorrichtung (1), einen Elektrodenkörper (10), der mit einem Generator (2) verbindbar ist, und ein Elektrodenende (11), das distal mit dem Elektrodenkörper (10) verbunden ist, umfassend, wobei das Elektrodenende (11) einen Träger (14) mit mindestens einer flachen Oberfläche und eine Vielzahl von Elektrodenelementen (12), die an der mindestens einen flachen Oberfläche des Trägers (14) befestigt sind, umfasst, wobei die Elektrodenelemente (12) durch das Verfahren nach einem der Ansprüche 1 bis 14 an dem Träger (14) befestigt werden.

## Revendications

1. Procédé de production d'un dispositif d' électrode implantable (1) comprenant un corps d'électrode (10) pouvant être connecté à un générateur (2) et une extrémité d'électrode (11) connectée de manière distale au corps d'électrode (10), l'extrémité d'électrode (11) comprenant un support électriquement isolant (14) et au moins un élément d'électrode (12) attaché au support (14), dans lequel le support (14) comprend au moins une surface plane, le procédé comprenant les étapes consistant à : fournir un support électriquement isolant (14), fournir l'au moins un élément d'électrode (12) électriquement conducteur, et attacher l'au moins un élément d'électrode (12) à l'au moins une surface plane du support (14),
dans lequel
pour attacher l'au moins un élément d'électrode (12) au support (14), l'au moins élément d'électrode (12) et/ou le support (14) sont chauffés et l'au moins un élément d'électrode (12) est pressé contre le support (14).

2. Procédé selon la revendication 1, dans lequel le support (14) est fabriqué en un matériau thermoplastique.

3. Procédé selon la revendication 1 ou 2, dans lequel le support (14) est fabriqué en un matériau comprenant du polyuréthane.

4. Procédé selon l'une des revendications 1 à 3, dans lequel pour attacher l'au moins un élément d'électrode (12) au support (14), l'au moins un élément d'électrode (12) et/ou le support (14) sont chauffés jusqu'à une température supérieure à 100 °C, préférentiellement supérieure à 150 °C, plus préférentiellement supérieure à 180 °C.

5. Procédé selon l'une des revendications précédentes, dans lequel l'au moins un élément d'électrode (12) est placé sur un côté supérieur (140) du support (14).

6. Procédé selon la revendication 5, dans lequel l'au moins un élément d'électrode (12) est pressé contre le support (14) de façon telle qu'un côté supérieur (123) de l'au moins un élément d'électrode (12) est aligné avec le côté supérieur (140) du support (14).

7. Procédé selon l'une des revendications précédentes, dans lequel une ligne d'alimentation électrique (13) est connectée à l'au moins un élément d'électrode (12) avant que l'au moins un élément d'électrode (12) ne soit attaché au support (14).

8. Procédé selon la revendication 7, dans lequel la ligne d'alimentation (13) connectée à l'au moins un élément d'électrode (12) est attachée au support (14) conjointement à l'au moins un élément d'électrode (12).

9. Procédé selon l'une des revendications précédentes, dans lequel l'au moins un élément d'électrode (12) a au moins un élément de fixation (120) qui est pressé dans le support (14) lorsque l'au moins un élément d'électrode (12) est attaché au support (14).

10. Procédé selon l'une des revendications précédentes, dans lequel l'au moins un élément d'électrode (12) a une broche (121) qui, lorsque l'au moins un élément d'électrode (12) est attaché au support (14), est pressée dans le support (14) de manière à ce que la broche (121) pénètre un côté inférieur (141) orienté dans la direction opposée à un côté supérieur (140) du support (14).

11. Procédé selon la revendication 10, dans lequel après que l'au moins un élément d'électrode (12) a été attaché au support (14), une ligne d'alimentation électrique (13) est connectée à la broche (121).

12. Procédé selon l'une des revendications précédentes, dans lequel le support (14) est au moins partiellement entouré d'un boîtier (15) après l'application de l'au moins un élément d'électrode (12).

13. Procédé selon la revendication 12, dans lequel le boîtier (15) est constitué d'un matériau à base de silicone.

14. Procédé selon l'une des revendications précédentes, dans lequel une pluralité d'éléments d'électrode (12) sont attachés au support (14).

15. Dispositif d'électrode implantable (1) comprenant un corps d'électrode (10) pouvant être connecté à un générateur (2) et une extrémité d'électrode (11) connectée de manière distale au corps d'électrode (10), dans lequel l'extrémité d'électrode (11) comprend un support (14) ayant au moins une surface plane et une pluralité d'éléments d'électrode (12) attachés à l'au moins une surface plane du support (14), dans lequel les éléments d'électrode (12) sont attachés au support (14) par le procédé selon l'une des revendications 1 à 14.
